# EUROPEAN PATENT SPECIFICATION

(11) **EP 3 747 407 B1**
(45) Date of publication and mention of the grant of the patent: **07.05.2025**
(21) Application number: 19747479.4
(22) Date of filing: 16.01.2019
(51) Int. Cl.: A61F 2/82, B29C 63/42

(54) **PROTECTION DEVICE FOR STENT**
SCHUTZVORRICHTUNG FÜR STENT
DISPOSITIF DE PROTECTION POUR ENDOPROTHÈSE

(30) Priority: 02.02.2018 CN 201810106942
(43) Date of publication of application: 09.12.2020
(73) Proprietor: Shanghai MicroPort Medical (Group) Co., Ltd., Shanghai 201203 (CN)
(72) Inventor: LI, Dan, Shanghai 201203 (CN); PANG, Lu, Shanghai 201203 (CN); WANG, Xueqin, Shanghai 201203 (CN); LUO, Qiyi, Shanghai 201203 (CN)
(74) Representative: Patentship Patentanwaltsgesellschaft mbH
(86) International application number: PCT/CN2019/071966
(87) International publication number: WO 2019/149069

(56) References cited:
- CN-A- 105 764 451
- CN-A- 107 488 345
- US-A1- 2004 093 005
- US-A1- 2004 093 005
- US-A1- 2007 061 001
- US-A1- 2007 061 001
- US-A1- 2011 208 292
- US-A1- 2012 109 281
- US-A1- 2012 109 281
- US-A1- 2015 088 240

## Description

### TECHNICAL FIELD

The present invention relates to the technical field of medical instruments and, more specifically, to a protection device for a stent.

### BACKGROUND

Bioabsorbable (biodegradable) polymer coronary stent products can bring benefits to the luminal diameter of coronary arteries and are suitable for use in the treatment of ischemic heart disease caused by primary coronary abnormalities. Such a bioabsorbable polymer stent product mainly consists of a polymer stent, a drug coating and a balloon-expandable delivery system.

Stents are generally categorized into metal and polymer ones, and metal stents are further grouped into biodegradable and non-biodegradable. At present, most metal stents are non-biodegradable, while most polymer ones are biodegradable. In many therapeutic applications, once a commonly-used metal stent is implanted, it will permanently remain in the patient's body, posing long-term risks to the patient's health. By contrast, a polymer stent stays in the body only for a limited period of time during which the stent achieves its expected goal such as blood vessel patency or drug delivery.

After achieving its therapeutic goal, a biodegradable stent will degrade into absorbable and metabolizable products in the body environment and eventually disappear. Before a stent is delivered into a diseased blood vessel, it is configured in a squeezed state of being squeezed and secured onto the surface of a balloon. During use, the squeezed stent will be delivered along with the balloon to the diseased blood vessel, and the balloon is then inflated so that the stent expands synchronously with the balloon and finally presses against the vessel wall.

Metal stents have good shape stability. Once a metal stent is squeezed to a certain diameter, it will substantially maintain the same diameter from then on. Therefore, metal stent products are basically free of the risk of diametrical expansion over time from packaging to use. On the other hand, polymer stents are made of polymeric materials that are more or less resilient, so they tend to experience more significant radial resilience after being moved from the presser. As a result, polymer stents will expand in radial dimension over time after being squeezed. However, traditional protective sheaths of a single-layered design tend to be unable to provide sufficient resistance to such expansions for stent diameter maintenance. Therefore, there is a need to address the challenges associated with securing a polymer stent to a delivery balloon and maintaining a diametrical dimension thereof favorable to the delivery system up until the time when the stent system is delivered to a target site within a body.

Conventional stents such as that disclosed in US2012109281A1 require complicated operations and suffer from a lack of compatibility with surgical environments.

US 2007/061001 A1 discloses a device for protecting the surface of a coated stent during its packaging, shipping, subsequent removal from the packaging and handling prior to introduction into a guiding catheter. US 2015/0088240 A1 discloses protective sheaths for scaffolds and stents crimped to a delivery balloon.

Those skilled in the art have been long seeking solutions to the problem that conventional polymer stent products expand in radial dimension over time from packaging to use.

### SUMMARY OF THE INVENTION

The invention is set out in the appended set of claims.

It is an objective of the present invention to provide a protection device for stent and a medical device, which can be operated easily and radially restrain and protect a stent.

To solve the above technical problem, the present invention provides a protection device for stent, comprising a sheath, a first grip and a second grip, the sheath being configured to hold a stent, the first grip is configured to be able to be driven to move toward the second grip to cause a first portion of the sheath to move away from a second portion thereof, so that the stent is allowed to be removed from the sheath.

Optionally, in the protection device for stent, the first grip is formed by extending the first portion, and the second grip is formed by extending the second portion.

Optionally, in the protection device for stent, the first grip and the second grip are connected to each other by a connecting member.

Optionally, in the protection device for stent, the connecting member is a spring, a spring strip or a shaft.

Optionally, in the protection device for stent, further comprising a first fastener and a second fastener, the first fastener being connected to the first grip, the second fastener being connected to the second grip, the first fastener and the second fastener being configured to engage each other to limit relative position of the first grip to the second grip.

Optionally, in the protection device for stent, each of the first grip and the second grip is provided thereon with a number of anti-slipping means.

Optionally, in the protection device for stent, an end of the first portion away from the first grip is integral with an end of the second portion away from the second grip.

Optionally, in the protection device for stent, the first grip defines an axially-extending opening through which the second grip is inserted so that the second grip opposes the first grip.

Optionally, in the protection device for stent, the connecting member is a spring strip with two ends disposed close to respective centers of the first grip and the second grip.

According to the invention, in the protection device for stent, the first portion and/or the second portion comprise(s) a frame and elastomer filler that is filled in the frame and defines a cavity for receiving the stent.

Optionally, in the protection device for stent, further comprising a protective layer provided on an inner surface of the sheath.

Accordingly, the present invention further provides a medical device, comprising a stent squeezed within a balloon catheter, a coiled tube and a protection device for stent as defined above, the protection device being connected to the coiled tube.

Optionally, in the medical device, the coiled tube comprises an engagement tough configured to be received in the sheath so that the engagement tough and the first portion of the sheath together defines a cavity for receiving the stent, and wherein when the first portion and the second portion of the sheath are separated from each other, the protection device is removable from the coiled tube.

Optionally, in the medical device, the protection device further comprises fasteners that are secured to an outer surface of the coiled tube or clamped onto an inner surface of the coiled tube by elastic deformation.

Optionally, in the medical device, the protection device further comprises a protective layer provided on an inner surface of the sheath.

In the protection device for stent and the medical device provided in the present invention, the protection device for stent mainly includes the sheath and the first and second grips. The sheath is configured to hold a stent, and the first grip is able to be driven to move toward the second grip so that the first portion of the sheath is caused to move away from the second portion thereof, allowing removal of the stent from the sheath. The protection device for stent according to the present invention can be operated by only one hand to cause the first grip to move toward the second grip, resulting in the separation of the sheath's first portion from the sheath's second portions. This easy operation results in a significant improvement in operating efficiency.

### BRIEF DESCRIPTION OF THE DRAWINGS

Figures 1 to 9 do not represent embodiments of the invention.
Fig. 1 is a structural schematic of a protection device for stent.
Fig. 2 is a side view of the protection device for stent of Fig. 1.
Fig. 3 is a schematic illustration of a first structural variant of a protection device for stent.
Fig. 4 is a structural schematic of a coiled tube.
Fig. 5 is a structural schematic of the protection device for stent of the Fig. 3 assembled with the coiled tube.
Fig. 6 is a schematic illustration of a second structural variant of the protection device for stent.
Fig. 7 is a side view of the protection device for stent of Fig. 6.
Fig. 8 is a schematic illustration of a third structural variant of the protection device for stent.
Fig. 9 is a side view of the protection device for stent of Fig. 8.
Fig. 10 is a schematic illustration showing a side view of a fourth structural variant of the protection device for stent according to the present invention.
Fig. 11 is a structural schematic of the protection device for stent of Fig. 10, which has been operated by one hand to result in a diametrical expansion in a cavity that restrains resilience of a stent.
Fig. 12 is a side view of the protection device for stent of Fig. 10 additionally provided with fasteners.
Fig. 13 is a structural schematic of the protection device for stent assembled with a stent and a balloon catheter.

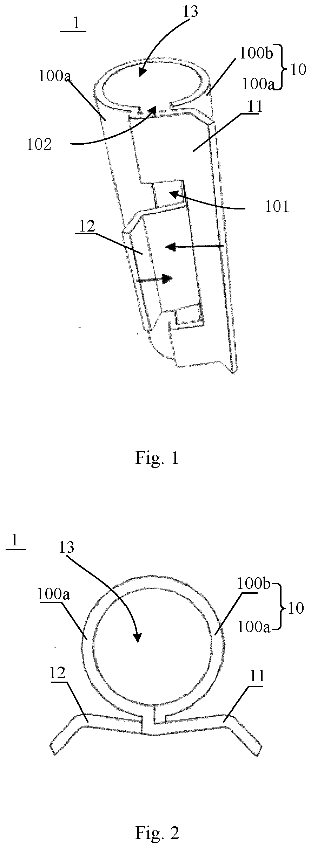

In Figs. 1 to 2, 1 denotes a protection device for stent; 10, a sheath; 100a, a first portion; 100b, a second portion; 101, an opening; 102, a slot; 11, a first grip; 12, a second grip; and 13, a cavity.

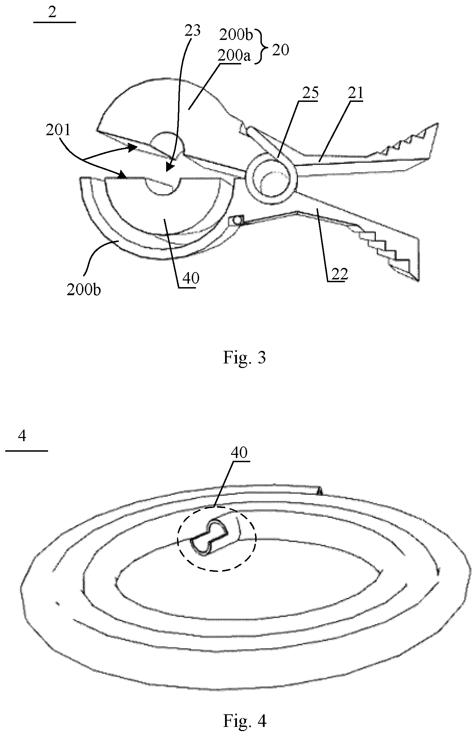

In Figs. 3 to 9 and 13, 2 denotes a protection device for stent; 20, sheath; 200a, a first portion; 200b, a second portion; 201, a slit; 21, a first grip; 22, a second grip; 23, a cavity; 24, a protective layer; 25, a connecting member; 26, a first fastener; 27, a second fastener; 4, a coiled tube; and 40, an engagement tough.

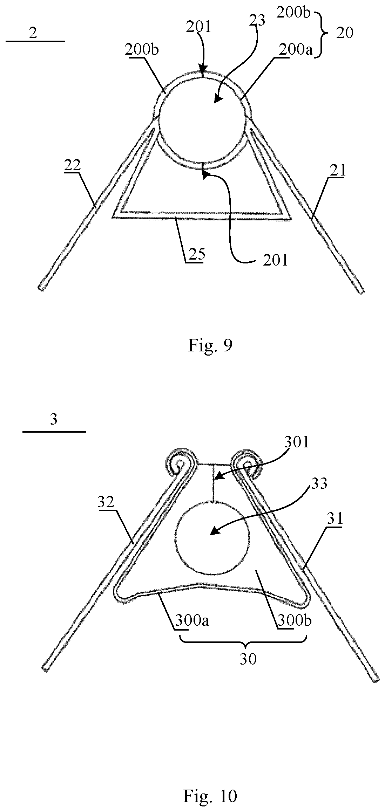

In Figs. 10 to 13, 3 denotes a protection device for stent; 30, a sheath; 300a, a frame; 300b, elastomer filler; 300c, a fastener; 301, a slit; 31, a first grip; 32, a second grip; 33, a cavity; 34, a protective layer; 5, a stent; and 6, a balloon catheter.

### DETAILED DESCRIPTION

The protection device for stent proposed herein will be described in greater detail below by way of particular examples with reference to the accompanying drawings. Features and advantages of the invention will be more apparent from the following detailed description, and from the appended claims. Note that the figures are provided in a very simplified form not necessarily drawn to scale, with the only intention to facilitate convenience and clarity in explaining the examples. In particular, as the figures tend to have distinct emphases, they are often drawn to different scales.

In this specification, when reference is made to a "stent", this term will refer to a squeezed stent. That is, a "squeezed stent" will be referred to as a "stent" hereinafter for short. The protection device for stent of the present invention can protect a squeezed stent while maintaining it to a diametrical dimension in favor of a delivery system.

The protection device for stent includes a sheath, a first grip and a second grip. The sheath is configured to hold a stent, and the first grip can be driven to move toward the second grip so that a first portion of the sheath is caused to move away from a second portion thereof, allowing removal of the stent from the sheath.

The first portion and/or the second portion include(s) a frame and elastomer filler that is filled in the frame and define a cavity for receiving the stent. The protection device for stent of the present invention will be better understood from the following description of a few specific examples.

### <Embodiment 1>

Reference is now made to Fig. 1, a structural schematic of a protection device for stent according to the present embodiment, and to Fig. 2, a side view of the protection device for stent of Fig. 1. As illustrated in Figs. 1 and 2, the protection device for stent 1 includes a sheath 10, a first grip 11 and a second grip 12. The sheath 10 is configured to hold a stent and includes first and second portions connected to each other. The first grip 11 can be driven to move toward the second grip 12 so that the first portion of the sheath 10 is caused to move away from the second portion thereof, allowing removal of the stent from the sheath 10. The first grip 11 is formed of an extension of the first portion of the sheath 10, whilst the second grip 12 is formed of an extension of the second portion of the sheath 10. The first and second portions are integral with each other at their respective sides away from the first and second grips, respectively. The first grip 11 defines an axially-extending opening 101, through which the second grip 12 is inserted and opposes the first grip 11. In another embodiment, the second grip 12 may define an axially-extending opening, through which the first grip 11 is inserted and opposes the second grip 12.

In order to increase friction on the first and second grips 11, 12 during use and obtain improved operational stability, a number of anti-slipping means are preferably provided on the first and second grips 11, 12 so that some of them are arranged on a side of the first grip 11 facing away from the second grip 12, and the rest on a side of the second grip 12 facing away from the first grip 11.

With continued reference to Fig. 1, in particular, the sheath 10 may define an axially-extending slot 102 having two edges opposing each other along a circumferential direction of the sheath 10 (and respectively forming an end of the first portion close to the first grip and an end of the second portion close to the second grip). The first and second grips 11, 12 may be connected respectively to these edges, with one being inserted into the other, so that they bridge the slot and define (delimit), together with the sheath 10, a cavity 13 that retrains the stent's resilience.

The cavity 13 delimited by the sheath 10, the first grip 11 and the second grip 12 restrains the stent's resilience by providing radial compressive forces, and the bridging of the slot 102 by the first and second grips 12 that are inserted one into the other provides radial support for maintain a circular cross-section of the stent. This effectively prevents radial resilience of the stent so that, when the stent is squeezed and secured to a balloon in a delivery system, it can maintain a diametrical dimension favorable to the delivery system (i.e., a diameter as when it was squeezed) up until the time when the stent system is delivered to a target site within a body. In addition, in the protection device for stent 1 of this embodiment, in addition to being inserted one into the other to bridge the slot 102 and delimit the cavity 13 that restrains the stent's resilience together with the sheath 10, the first and second grips 11, 12 are also allowed to each have a significantly reduced width (perpendicular to an axis of the protection device 1) so as to allow the protection device to be loaded in (i.e., accommodated within) another structure.

The sheath 10 and the first and second grips 11, 12 may be formed by forging and cutting a metal sheet, or by molding a polymeric material. The metal sheet is preferably made of alloy(s) with good biocompatibility, such as at least one of a stainless steel, a nickel-titanium alloy and a magnesium alloy. The polymeric material is preferably a polymeric material with good biocompatibility containing, for example, at least one of PTFE, Pebax, polyethylene, polycarbonate, polyamide and nylon. The sheath 10 may appear as a hollow tube, and the stent is held therein in a squeezed state where its outer diameter is equal to an inner diameter of the sheath 10. In addition, the sheath 10 may have a length that is greater than or equal to a length of the squeezed stent so that it is able to entirely cover an outer surface of the stent and apply a constraining force radially to the squeezed stent, thus better restraining the stent's radial resilience.

In order for better understanding the working principles of the protection device for stent according to the present invention, the operation of the protection device for stent 1 of this embodiment will be described below in detail with reference to Figs. 1 and 2.

First of all, in step S11, external forces are exerted on the first and second grips 11, 12 so that the first grip 11 moves toward the second grip 12, causing the free ends of the first and second portions of the sheath 10 to move away from each other (manifested as an expansion of the slot 102 and a diametrical expansion of the cavity 13 that restrains the stent's resilience).

Next, in step S12, the stent is removed from the sheath 10.

Specifically, for example, this can be accomplished by only one hand of an operator. The operator may press the first and second grips 11, 12, with the thumb and index finger, respectively, in the directions indicated by the arrows in the Fig. 1. Under the pressure, the first grip 11 will move toward the second grip 12, causing the free ends of the first and second portions of the sheath 10 to move away from each other. This is manifested as movements of the two ends of the slot 102 opposing each other in the circumferential direction of the sheath 10 in the directions opposite to those indicated by the arrows, which results in an expansion of the slot 102 and a diametrical expansion of the cavity 13 that restrains the stent's resilience, making it possible to release the stent from constraints of the cavity 13 and thus from the sheath 10 in a contactless and frictionless manner.

### < Embodiment 2>

A protection device for stent according to a second embodiment is structurally different from that of Embodiment 1 in further including a connecting member, in addition to a sheath, a first grip and a second grip. The connecting member connects the first and second grips to each other. In this embodiment, the connecting member is a metal member.

Reference is now made to Fig. 3, a schematic illustration of a first structural variant of the protection device for stent according to this embodiment. As shown in Fig. 3, the sheath 20 is split by a slit 201 into a first portion 200a and a second portion 200b that are separable from each other. The sheath 20 appears as a hollow tube and has a length that is greater than or equal to a length of a squeezed stent load therein, so that it is able to entirely cover an outer surface of the stent and apply a constraining force radially to the squeezed stent, thus better restraining the stent's radial resilience. In this way, when the stent is squeezed and secured to a balloon in a delivery system, it can maintain a diametrical dimension favorable to the delivery system up until the time when the stent system is delivered to a target site within a body.

With continued reference to Fig. 3, in the protection device for stent 2, the connecting member 25 connecting the first and second grips 21, 22 is preferably a spring tending to resist the pressure force that would cause opposing movements of the first and second grip 21, 22 and maintain the connection between the first and second portions 200a, 200b. This can ensure the stability of the cavity 23 that restrains the stent's resilience. When the pressure force is greater than the resistance threshold of the spring, the first and second portions 200a, 200b will move away from each other, leading to a diametrical expansion of the cavity 23 that restrains the stent's resilience.

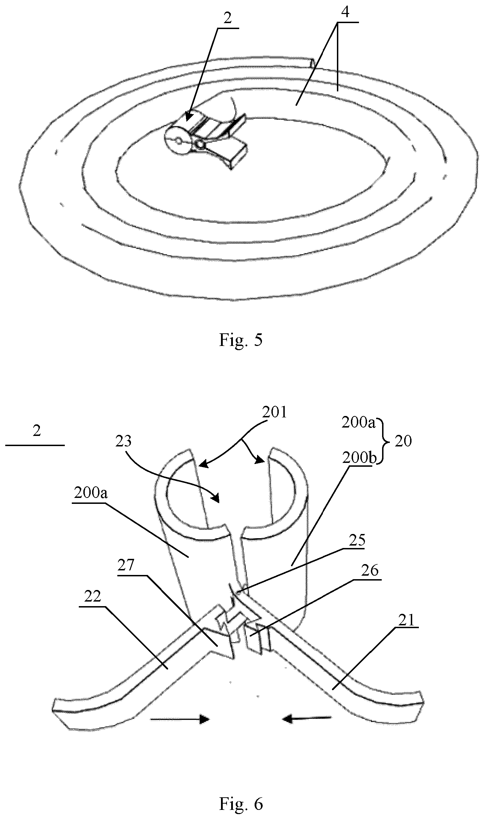

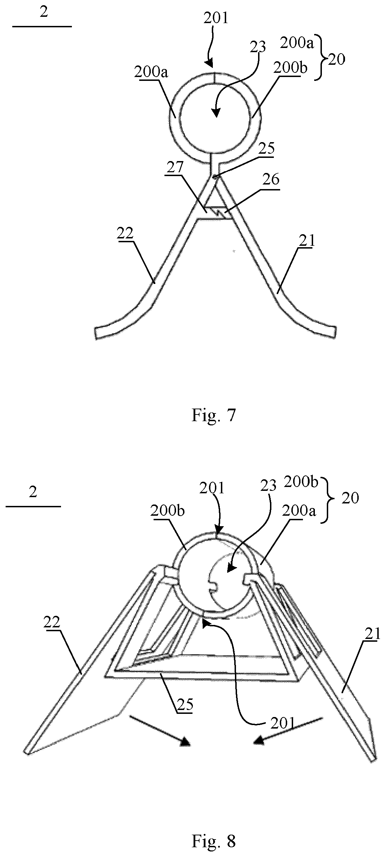

The connecting member 25 is not limited to being a spring, because it can be any one of multiple structures including, for example, a spring strip or a shaft, as in particular shown in Figs. 6 to 9. Reference is now made to Fig. 6, a schematic illustration of a second structural variant of the protection device for stent according to this embodiment, and to Fig. 7, a side view of the protection device for stent of Fig. 6. The protection device for stent 2 shown in Figs. 6 and 7 differs from that in Fig. 3 in the structure of the connecting member 25. As shown in Figs. 6 and 7, the connecting member 25 is a shaft such as a pin shaft. The first grip 21 is connected to the first portion 200a to form a first piece, and the second grip 22 is connected to the second portion 200b to form a second piece. The shaft extends through an overlap between the first and second pieces. In order for ensure robustness of the protection device, the protection device for stent 2 further includes a first fastener 26 and a second fastener 27. The first fastener 26 is connected to the first grip 21, while the second fastener 27 is connected to the second grip 22. When the first fastener 26 engages the second fastener 27, the first and second grips 21, 22 are limited in position relative to each other, with the first and second portions 200a, 200b being connected together.

Reference is now made to Fig. 8, a schematic illustration of a third structural variant of the protection device for stent according to this embodiment, and to Fig. 9, a side view of the protection device for stent of Fig. 8. The protection device for stent 2 shown in Figs. 8 and 9 differs from those in Fig. 3 and in Figs. 6 and 7 in the overall structure of the protection device and in the structure of the connecting member 25. As shown in Figs. 8 and 9, the connecting member 25 is a spring strip with ends disposed close to respective centers of the first and second grips. In this embodiment, the spring strip is curved and defines an opening in which the sheath 20 is secured. In particular, as shown in Fig. 9, the opening has two edges fixedly connected respectively to the first and second portions 200a, 200b. The first and second portions 200a, 200b are axisymmetric, and the two edges of the opening are further fixedly connected respectively to the first and second grips 21, 22. The first and second grips 21, 22 are axisymmetric. In this way, the spring strip provides a resistance force in response to the opposing movements of the first and second grip 21, 22, causing an expansion of the opening of the spring strip. As a result, the first and second portions 200a, 200b moves away from each other, and the cavity 23 that restrains the stent's resilience experiences a diametrical expansion. Preferably, the spring strip is C-shaped.

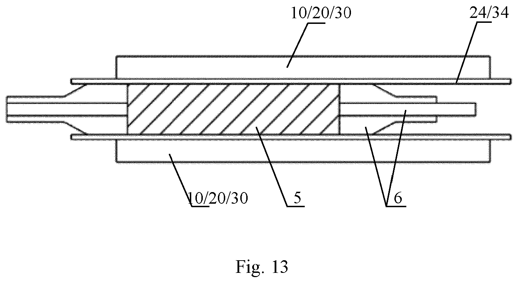

Reference is now made to Fig. 13, a structural schematic of the protection device for stent according to this embodiment assembled with a stent and a balloon catheter. As shown in Fig. 13, in order to avoid damage to a coating on the surface of the stent 5 during the removal of the stent from the sheath 20, the protection device according to this embodiment may further include a protective layer 24 disposed on an inner surface of the sheath 20 (i.e., surrounding an outer surface of the stent 5). The protective layer 24 can not only provide protection to the stent 5, but also allows the sheath 20 to apply a higher preload to the stent 5. Preferably, the sheath 20 is a metal sheath or a polymer sheath, with the first and second grips 21, 22 being both metal grips, and with the protective layer 24 being a polymer film wrapping the outer surface of the stent and insulating it from the sheath.

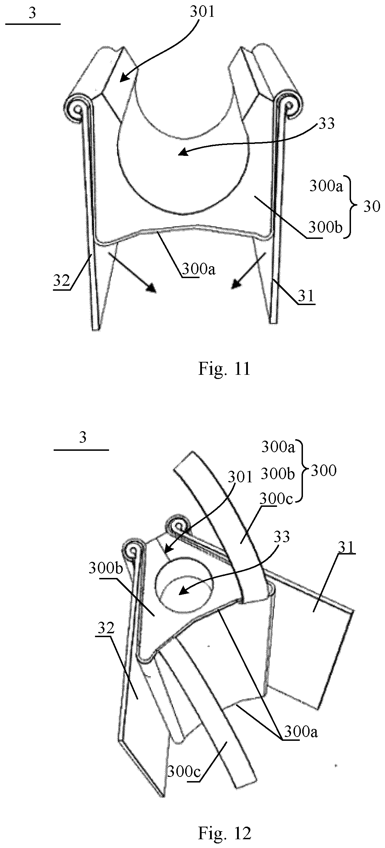

Reference is now made to Fig. 10, a schematic illustration of a fourth structural variant of the protection device for stent according to this embodiment. The protection device for stent of Fig. 10 differs from that of Fig. 8 in (i) omitting the connecting member, with the sheath providing the functions of the connecting member instead and (ii) differences in the structures of the first and second portions. As shown in Fig. 10, the sheath 30 of the protection device for stent 3 is split by a slit 301 into first and second portions with respective ends separable from each other. The first and second portions are integral with each other at the respective other ends. That is, the slit 301 extends from an outer surface of the sheath 30 radially into the cavity 33. The first and second portions include a frame 300a and elastomer filler 300b that is filled within the frame 300a and defines the cavity for receiving the stent. The first and second grips 31, 32 are disposed on opposing sides of the frame 300a. As shown in Fig. 11, in the process that the sheath 30 resists the pressure force on the first and second grips 31, 32, the sheath 30 will elastically deform, causing a diametrical expansion of the cavity 33 and thus allowing removal of the stent from the sheath 30.

As shown in Fig. 13, in order to avoid damage to a coating on the surface of the stent 5 during the removal of the stent from the sheath 30, the protection device for stent 3 according to this embodiment may further include a protective layer 34 disposed on an inner surface of the sheath 30 (i.e., surrounding an outer surface of the stent 5). The protective layer 34 can not only provide protection to the stent 5, but also allows the sheath 30 to apply a higher preload to the stent 5.

Referring to Fig. 12, on the basis of the structure shown in Fig. 10, the protection device for stent 3 may further include fasteners 300c enabling engagement with other components. Each fastener 300c can be structured as needed, and the present invention is not limited to any particular structure thereof.

### <Embodiment 3>

Referring to Figs. 4, 5 and 13, in an embodiment, there is provided a medical device including a stent 5 squeezed within a balloon catheter 6, a coiled tube 4 and any of the protection devices for stent as described in Embodiment 1 and 2. The protection device for stent is coupled to the coiled tube 4. In the case shown in Fig. 5, the medical device includes the protection device for stent 2 shown in Fig. 3.

With combined reference to Figs. 3 and 4, the coiled tube 4 includes an engagement tough 40 configured to be received in the sheath 20 so that the coiled tube 4 defines the stent cavity 23 together with the first portion 200a of the sheath 20. When the first and second portions 200a, 200b of the sheath are separated from each other, the protection device for stent 2 can be removed from the coiled tube 4.

The engagement of the protection device for stent with the coiled tube is not limited to being accomplished by the structure shown in Fig. 3 in which the engagement tough 40 and the first portion 200a of the sheath 20 together delimits the stent cavity. Alternatively, a groove that can engage the engagement tough 40 (the groove may be complementary to the engagement tough in cross-sectional shape so that the coiled tube can be assembled with the sheath when the engagement tough is engaged in the groove) may be formed in the first or second portion so that the first and second portions together delimit the stent cavity.

Referring to Fig. 12, the protection device for stent may further include fasteners 300c, which are fastened to an outer surface of the coiled tube or clamped onto an inner surface of the coiled tube by elastic deformation.

The embodiments disclosed herein are described in a progressive manner, with the description of each embodiment focusing on its differences from others. Reference can be made between the embodiments for their identical or similar parts. Since the medical device of Embodiment 3 employs a protection device for stent of Embodiment 1 or 2, it is described relatively briefly, and reference can be made to the related description of Embodiment 1 or 2 for more details in Embodiment 3. As the methods described in the embodiments correspond to the respective structures in the embodiments, they are described relatively briefly, and reference can be made to the related description of the structures for more details in the methods.

In summary, in the protection device for stent provided in the present invention, the protection device for stent mainly includes the sheath and the first and second grips. The sheath is configured to hold a stent, and the first grip is able to be driven to move toward the second grip so that the first portion of the sheath is caused to move away from the second portion thereof, allowing removal of the stent from the sheath. The protection device for stent can be operated by only one hand to cause the first grip to move toward the second grip, resulting in the separation of the sheath's first portion from the sheath's second portions. This easy operation results in a significant improvement in operating efficiency. Further, a diametrical expansion resulting from the opposing movements of the first and second grips allows the stent to be removed from the protection device without sliding contact between them. This can effectively avoid damage to the stent or its surface coating. Furthermore, the fasteners can provide stable support to the sheath for a long term, thus avoiding radial resilience of the stent due to deformation of the sheath during storage.

The description presented above is merely that of a few preferred embodiments of the present invention and does not limit the scope thereof in any sense. Any and all changes and modifications made by those of ordinary skill in the art based on the above teachings fall within the scope as defined in the appended claims.

## Claims

1. A protection device (1) for stent, comprising a sheath (10, 20, 30), a first grip (11, 21, 31) and a second grip (12, 22, 32), the sheath (10, 20, 30) being configured to hold a stent, the first grip (11, 21, 31) is configured to be able to be driven to move toward the second grip (12, 22, 32) to cause a first portion (100a, 200a) of the sheath (10, 20, 30) to move away from a second portion (100b, 200b) thereof, so that the stent is allowed to be removed from the sheath (10, 20, 30);
wherein the first portion (100a, 200a) and the second portion (100b, 200b) form a frame (300a), and an elastomer filler (300b) is filled in the frame (300a) and defines a cavity (33) for receiving the stent.

2. The protection device (1) for stent of claim 1, wherein the first grip (11, 21, 31) is formed of an extension of the first portion (100a, 200a), and the second grip (12, 22, 32) is formed of an extension of the second portion (100b, 200b).

3. The protection device (1) for stent of claim 2, wherein the first grip (11, 21, 31) and the second grip (12, 22, 32) are connected to each other by a connecting member (25).

4. The protection device (1) for stent of claim 3, wherein the connecting member (25) is a spring, a spring strip or a shaft.

5. The protection device (1) for stent of claim 4, further comprising a first fastener (26) and a second fastener (27), the first fastener (26) being connected to the first grip (11, 21, 31), the second fastener (27) being connected to the second grip (12, 22, 32), the first fastener (26) and the second fastener (27) being configured to engage each other to limit relative position of the first grip (11, 21, 31) to the second grip (12, 22, 32).

6. The protection device (1) for stent of claim 1, wherein each of the first grip (11, 21, 31) and the second grip (12, 22, 32) is provided thereon with a number of anti-slipping means.

7. The protection device (1) for stent of claim 2, wherein an end of the first portion (100a, 200a) away from the first grip (11, 21, 31) is integral with an end of the second portion (100b, 200b) away from the second grip (12, 22, 32).

8. The protection device (1) for stent of claim 7, wherein the first grip (11) defines an axially-extending opening (101) through which the second grip (12) is inserted so that the second grip (12) opposes the first grip (11).

9. The protection device (1) for stent of claim 4, wherein the connecting member (25) is a spring strip with two ends disposed close to respective centers of the first grip (21) and the second grip (22).

10. The protection device (1) for stent of claim 1, further comprising a protective layer (24, 34) provided on an inner surface of the sheath (20, 30).

11. A medical device, comprising a stent (5) squeezed within a balloon catheter (6), a coiled tube (4) and a protection device (1) for stent as defined in any one of claims 1 to 9, the protection device (1) being connected to the coiled tube (4).

12. The medical device of claim 11, wherein the coiled tube (4) comprises an engagement tough (40) configured to be received in the sheath (10, 20, 30) so that the engagement tough (40) and the first portion (100a, 200a) of the sheath (10, 20, 30) together defines a cavity (13, 23, 33) for receiving the stent (5), and wherein when the first portion (100a, 200a) and the second portion (100b, 200b) of the sheath (10, 20, 30) are separated from each other, the protection device (1) is removable from the coiled tube (4).

13. The medical device of claim 11, wherein the protection device (1) further comprises fasteners (300c) that are secured to an outer surface of the coiled tube (4) or clamped onto an inner surface of the coiled tube (4) by elastic deformation.

14. The medical device of claim 11, wherein the protection device (1) further comprises a protective layer (24, 34) provided on an inner surface of the sheath (20, 30).

## Patentansprüche

1. Eine Schutzvorrichtung (1) für einen Stent, die eine Hülle (10, 20, 30), einen ersten Griff (11, 21, 31) und einen zweiten Griff (12, 22, 32) umfasst, wobei die Hülle (10, 20, 30) ausgebildet ist, einen Stent zu halten , und der erste Griff (11, 21, 31) ausgebildet ist, angetrieben werden zu können, um sich in Richtung des zweiten Griffs (12, 22, 32) zu bewegen, um zu bewirken, dass sich ein erster Abschnitt (100a, 200a) der Hülle (10, 20, 30) von einem zweiten Abschnitt (100b, 200b) derselben weg bewegt, sodass der Stent aus der Hülle (10, 20, 30) entfernt werden kann;
wobei der erste Abschnitt (100a, 200a) und der zweite Abschnitt (100b, 200b) einen Rahmen (300a) bilden und ein Elastomerfüllstoff (300b) in den Rahmen (300a) gefüllt ist und einen Hohlraum (33) zur Aufnahme des Stents definiert.

2. Die Schutzvorrichtung (1) für einen Stent nach Anspruch 1, wobei der erste Griff (11, 21, 31) aus einer Verlängerung des ersten Abschnitts (100a, 200a) gebildet ist, und der zweite Griff (12, 22, 32) aus einer Verlängerung des zweiten Abschnitts (100b, 200b) gebildet ist.

3. Die Schutzvorrichtung (1) für einen Stent nach Anspruch 2, wobei der erste Griff (11, 21, 31) und der zweite Griff (12, 22, 32) durch ein Verbindungselement (25) miteinander verbunden sind.

4. Die Schutzvorrichtung (1) für einen Stent nach Anspruch 3, wobei das Verbindungselement (25) eine Feder, ein Federstreifen oder ein Schaft ist.

5. Die Schutzvorrichtung (1) für einen Stent nach Anspruch 4, die ferner ein erstes Befestigungselement (26) und ein zweites Befestigungselement (27) umfasst, wobei das erste Befestigungselement (26) mit dem ersten Griff (11, 21, 31) verbunden ist, wobei das zweite Befestigungselement (27) mit dem zweiten Griff (12, 22, 32) verbunden ist, wobei das erste Befestigungselement (26) und das zweite Befestigungselement (27) ausgebildet sind, ineinander zu greifen, um die relative Position des ersten Griffs (11, 21, 31) zu dem zweiten Griff (12, 22, 32) zu begrenzen.

6. Die Schutzvorrichtung (1) für einen Stent nach Anspruch 1, wobei der erste Griff (11, 21, 31) und der zweite Griff (12, 22, 32) jeweils mit einer Anzahl von Antirutschmitteln versehen sind.

7. Die Schutzvorrichtung (1) für einen Stent nach Anspruch 2, wobei ein von dem ersten Griff (11, 21, 31) entferntes Ende des ersten Abschnitts (100a, 200a) mit einem von dem zweiten Griff (12, 22, 32) entfernten Ende des zweiten Abschnitts (100b, 200b) einstückig ist.

8. Die Schutzvorrichtung (1) für einen Stent nach Anspruch 7, wobei der erste Griff (11) eine sich axial erstreckende Öffnung (101) definiert, durch die der zweite Griff (12) so eingeführt wird, dass der zweite Griff (12) dem ersten Griff (11) gegenüberliegt.

9. Die Schutzvorrichtung (1) für einen Stent nach Anspruch 4, wobei das Verbindungselement (25) ein Federstreifen ist, dessen zwei Enden nahe an den jeweiligen Mittelpunkten des ersten Griffs (21) und des zweiten Griffs (22) angeordnet sind.

10. Die Schutzvorrichtung (1) für einen Stent nach Anspruch 1, die ferner eine Schutzschicht (24, 34) umfasst, die auf einer Innenfläche der Hülle (20, 30) vorgesehen ist.

11. Eine medizinische Vorrichtung, die einen innerhalb eines Ballonkatheters (6) gequetschten Stent (5), einen gewickelten Schlauch (4) und eine Schutzvorrichtung (1) für einen Stent gemäß einem der Ansprüche 1 bis 9 umfasst, wobei die Schutzvorrichtung (1) mit dem gewickelten Schlauch (4) verbunden ist.

12. Die medizinische Vorrichtung nach Anspruch 11, wobei der gewickelte Schlauch (4) eine Eingriffsmulde (40) umfasst, die ausgebildet ist, in der Hülle (10, 20, 30) aufgenommen zu werden, so dass die Eingriffsmulde (40) und der erste Abschnitt (100a, 200a) der Hülle (10, 20, 30) zusammen einen Hohlraum (13, 23, 33) zur Aufnahme des Stents (5) definieren, und wobei die Schutzvorrichtung (1) von dem gewickelten Schlauch (4) abnehmbar ist, wenn der erste Abschnitt (100a, 200a) und der zweite Abschnitt (100b, 200b) der Hülle (10, 20, 30) voneinander getrennt sind.

13. Die medizinische Vorrichtung nach Anspruch 11, wobei die Schutzvorrichtung (1) ferner Befestigungselemente (300c) umfasst, die an einer Außenfläche des gewickelten Schlauches (4) befestigt sind oder durch elastische Verformung an einer Innenfläche des gewickelten Schlauches (4) festgeklemmt sind.

14. Die medizinische Vorrichtung nach Anspruch 11, wobei die Schutzvorrichtung (1) ferner eine Schutzschicht (24, 34) umfasst, die auf einer Innenfläche der Hülle (20, 30) vorgesehen ist.

## Revendications

1. Dispositif de protection (1) pour stent, comprenant une gaine (10, 20, 30), une première poignée (11, 21, 31) et une seconde poignée (12, 22, 32), la gaine (10, 20, 30) étant conçue pour maintenir un stent, la première poignée (11, 21, 31) étant conçue pour pouvoir être déplacée vers la seconde poignée (12, 22, 32) pour provoquer une première partie (100a, 200a) de la gaine (10, 20, 30) pour s'éloigner d'une deuxième partie (100b, 200b) de celui-ci, de sorte que le stent puisse être retiré de la gaine (10, 20, 30) ;
dans lequel la première partie (100a, 200a) et la seconde partie (100b, 200b) forment un cadre (300a), et une charge élastomère (300b) est remplie dans le cadre (300a) et définit une cavité (33) pour recevoir le stent.

2. Dispositif de protection (1) pour stent selon la revendication 1, dans lequel la première poignée (11, 21, 31) est formée d'une extension de la première partie (100a, 200a), et la seconde poignée (12, 22, 32) est formée d'une extension de la seconde partie (100b, 200b).

3. Dispositif de protection (1) pour stent selon la revendication 2, dans lequel la première poignée (11, 21, 31) et la seconde poignée (12, 22, 32) sont reliées l'une à l'autre par un élément de liaison (25).

4. Dispositif de protection (1) pour stent selon la revendication 3, dans lequel l'élément de liaison (25) est un ressort, une bande de ressort ou un arbre.

5. Dispositif de protection (1) pour stent selon la revendication 4, comprenant en outre une première attache (26) et une seconde attache (27) , la première attache (26) étant reliée à la première poignée (11, 21, 31), la seconde attache (27) étant reliée à la seconde poignée (12, 22, 32) , la première attache (26) et la seconde attache (27) étant reliées à la seconde poignée (12, 22, 32) étant configurés pour s'engager l'un dans l'autre afin de limiter la position relative de la première poignée (11, 21, 31) à la deuxième poignée (12, 22, 32).

6. Dispositif de protection (1) pour stent selon la revendication 1, dans lequel chacune des première (11, 21, 31) et seconde (12, 22, 32) poignées est pourvue d'un certain nombre de moyens antidérapants.

7. Dispositif de protection (1) pour stent selon la revendication 2, dans lequel une extrémité du premier portion (100a, 200a) à l'écart de la première poignée (11, 21, 31) est solidaire d'une extrémité de la deuxième partie (100b, 200b) à l'écart de la deuxième poignée (12, 22, 32).

8. Dispositif de protection (1) pour stent selon la revendication 7, dans lequel la première poignée (11) définit une ouverture s'étendant axialement (101) à travers laquelle la seconde poignée (12) est insérée de sorte que la seconde poignée (12) s'oppose à la première poignée (11).

9. Dispositif de protection (1) pour stent selon la revendication 4, dans lequel l'élément de connexion (25) est une bande à ressort avec deux extrémités disposées à proximité des centres respectifs de la première poignée (21) et de la seconde poignée (22).

10. Dispositif de protection (1) pour stent selon la revendication 1, comprenant en outre une couche protectrice (24, 34) prévue sur une surface intérieure de la gaine (20, 30).

11. Un dispositif médical, comprenant un stent (5) pressé Dans un cathéter à ballonnet (6), un tube spiralé (4) et un dispositif de protection (1) pour stent tel que défini dans l'une quelconque des revendications 1 à 9, le dispositif de protection (1) être relié au tube spiralé (4).

12. Dispositif médical selon la revendication 11, dans lequel le tube spiralé (4) comprend un élément d'engagement (40) configuré pour être reçu dans la gaine (10, 20, 30) de sorte que l'élément d'engagement (40) et la première partie (100a, 200a) de la gaine (10, 20, 30) définissent ensemble une cavité (13, 23, 33) pour recevoir le stent (5), et dans lequel lorsque la première partie (100a, 200a) et la seconde partie (100b, 200b) de la gaine (10, 20, 30) sont séparées l'une de l'autre, le dispositif de protection (1) est amovible du tube spiralé (4).

13. Dispositif médical selon la revendication 11, dans lequel le dispositif de protection (1) comprend en outre des éléments de fixation (300c) qui sont fixés à une surface extérieure du tube enroulé (4) ou serrés sur une surface intérieure du tube enroulé (4) par déformation élastique.

14. Dispositif médical selon la revendication 11, dans lequel le dispositif de protection (1) comprend en outre une couche protectrice (24, 34) prévue sur une surface intérieure de la gaine (20, 30).
